# EUROPEAN PATENT SPECIFICATION

(11) **EP 1 365 799 B1**
(45) Date of publication and mention of the grant of the patent: **07.09.2005**
(21) Application number: 01274060.1
(22) Date of filing: 15.02.2001
(51) Int. Cl.: A61K 39/00, A61K 39/205, C12N 1/00, C12N 15/00, C12N 15/47

(54) **A NOVEL VACCINE FORMULATION CONSISTING OF DNA VACCINE INACTIVATED VIRUS**
NEUE VAKZINFORMULIERUNG AUS DNA-VAKZINE-INAKTIVIERTEM VIRUS
NOUVELLE FORMULATION DE VACCIN CONSTITUEE D'UN VIRUS INACTIVE DE VACCIN ADN

(43) Date of publication of application: 03.12.2003
(73) Proprietor: The Registrar, Indian Institute of Science, Karnataka State (IN); Indian Immunologicals Limited, Andhra Pradesh (IN)
(72) Inventor: PUNDI, Narasimhan Rangarajan, Bangalore 560 079, Karnataka State (IN); VILLUPPANOOR, Alwar Srinivasan, Hyderabad 500 019, Andhra Pradesh (IN); SUBHABRATA, Biswas, "Sitanath Smriti", Calcutta 700 064, West Bengal (IN); GUDDETI, Sreenivasa Reddy, Hyderabad 500 019, Andhra Pradesh (IN)
(74) Representative: Kremer, Simon Mark
(86) International application number: PCT/IN2001/000018
(87) International publication number: WO 2002/078732

(56) References cited:
- WO-A1-90/00191
- WO-A1-97/23502
- US-A- 5 348 741
- DATABASE MEDLINE [Online] FODOR I. ET AL.: 'Gene immunization of mice with plasmid DNA expressing rabies virus glycoprotein', XP002970697 Retrieved from NCBI Database accession no. 11402706 & ACTA VET HUNG vol. 48, no. 2, 2000, pages 229 - 236

## Description

### TECHNICAL FIELD

The present invention relates to a rabies vaccine formulation for use in prophylactic or therapeutic immunization of vertebrates against rabies. The vaccine composition contains a minimum of two components, one of which is a deoxyribonucleotide (DNA) vaccine comprising of a DNA molecule that encodes a surface glycoprotein of the virus and the other component consisting of inactivated form of the virus. The protective immune responses induced by the administration of these two components together is superior to that induced by the administration of individual components alone. Thus, this invention can be used to develop a combination vaccine consisting of DNA vaccine and inactivated virus. In another aspect, this invention can be used to develop low cost inactivated virus-based vaccines that contain much lower amount of the said virus than that present in similar vaccines known in the prior art.

### BACKGROUND OF THE INVENTION

Ever since Edward Jenner first documented the successful vaccination strategy for small pox more than two hundred years ago, vaccine development has undergone dramatic changes. The first generation vaccines involved the use of attenuated, live or killed pathogens as vaccines and this mode of vaccination was primarily responsible for eradicating diseases such as polio, small pox etc. Rapid progress in animal cell culture technology led to development of cell culture-based vaccines, wherein the pathogenic organisms were cultured in large scale, purified, inactivated and used as vaccines. The use of killed or inactivated viruses as vaccines is widely practiced for diseases such as polio, rabies, measles etc. In this method of vaccination, the virus is presented to the immune system in a non-infective form so that the individual can mount an immune response against it. Killed or inactivated viruses provide protection by directly generating T-helper and humoral immune responses against the immunogens of the virus. However, in this type of vaccination, because the virus does not replicate or undergo an infective cycle, cell-mediated immune response mediated by cytotoxic T lymphocytes (CTLs) is not generated. In the absence of an efficient CTL response, these vaccines often do not confer complete protection against pathogen. Another major problem associated with killed or inactivated virus-based vaccines is their high cost of production. Although several such tissue culture-based inactivated virus vaccines are available for diseases such as rabies, the high cost of production of the virus at high titres using tissue culture methods renders these vaccines uneconomical in many parts of the world especially in the developing countries, where the demand for inactivated virus-based vaccines far exceeds the supply. Thus, a major draw back of these types of vaccines is their high cost of production and difficulty of producing them in large quantities. Strategies which decrease the quantity of inactivated virus in the vaccine formulation without compromising on vaccine potency can lower the cost of production of inactivated virus-based vaccines. Thus, there is a need to develop novel vaccines, which can overcome all or some of the drawbacks associated with inactivated virus-based vaccines.

The use of plasmid DNA as a vaccine assumes great significance since it can be produced at a very low cost and can be stored at room temperature. In the seminal study by Wolff *et al* of "plasmid or naked" DNA vaccination in vivo, it was shown that direct intramuscular inoculation of plasmid DNA encoding several different reporter genes could induce protein expression within the muscle cells (1). This study provided a strong basis for the notion that purified/ recombinant nucleic acids ("naked DNA") can be delivered *in vivo* and can direct protein expression. These observations were further extended in a study by Tang et al (2), who demonstrated that mice injected with plasmid DNA encoding hGH could elicit antigen-specific antibody responses. Subsequently, demonstrations by Ulmer *et al* (3) and Robinson *et al* (4) that DNA vaccines could protect mice or chickens, respectively, from influenza infection provided a remarkable example of how DNA vaccination could mediate protective immunity. The mouse study further documented that both antibody and CD8⁺ cytotoxic T-lymphocyte (CTL) responses were elicited (3,4), consistent with DNA vaccines stimulating both humoral and cellular immunity. This was followed by several reports which demonstrated the utility of DNA, vaccines to induce protective immune response against several infectious diseases including cancer in experimental models. The art is rich in literature on DNA vaccines as evident from several books (5-9) and review articles (10-38) which have been published on this subject. A web site devoted to DNA vaccines also provides valuable information on the progress made in this area (39). Further, guidelines for the use of DNA vaccines in animal and human clinical trials are also available (40). Other information that is helpful in understanding and practice of the DNA vaccine technology can be found in the patent Nos.US05580859, US05589466, US05620896, US05736524, US05939400, US05989553, US06063385, US06087341, US06090790, US06110898, US06156322, US05576196, US05707812, US5643578, US0557619, US0570781, US05916879, US05958895, US05830876, US05817637, US6133244, US6020319, US5593972, WO00/24428, WO99/51745, WO99/43841, WO99/388880, WO99/02132, WO98/14586, WO98/08947, WO98/04720, WO00/77188, WO00/77043, WO00/77218, WO0077188, WO00037649, WO00044406, WO09904009, WO00053019, WO00050044, WO00012127, WO09852603, WO9748370, and WO097730587. The vast literature available on DNA vaccines indicates that DNA vaccines hold a great deal of promise in the prevention and treatment of several infectious diseases including cancer. The general applicability of DNA vaccines to induce protective immunity has been well established in several animal models and this has led to phase I human clinical trials. Studies carried out in the last couple of years indicate that for certain diseases, DNA vaccination alone cannot produce the desired effect, but if used in combination with other prophylactic or therapeutic strategies can yield the desired results. One such strategy, referred to in the prior art as the 'prime-boost strategy' involves administration of a DNA vaccine to 'prime' the immune system and this is followed by the administration of recombinant protein or live attenuated vaccines or inactivated pathogen-based vaccines to 'boost' the immune system (41-59). In addition to the prime-boost strategy, several other strategies are described in the prior art for improving the potency of DNA vaccines. These include:
1. co-inoculation of multiple plasmids expressing different antigens of a pathogen or plasmids expressing multiple epitopes of different antigens of a pathogen (60-71).
2. co-inoculation of plasmids encoding cytokines or co-stimulatory molecules and plasmids expressing antigens of interest (72-94).
3. - Inclusion of certain compounds such as saponin, alum, chitosan, liposomes, cationic lipids etc., in the DNA vaccine preparations (95-106).
4. Inclusion of specific sequences referred to as the CpG motifs in the plasmid DNA (107-117).

Further, a variety of strategies on modification of plasmid vector and/or the gene encoding the antigen were shown to improve the potency of DNA vaccines (118-136). Thus, there is scope for the development of novel compositions that can improve the potency of DNA vaccines.

### SUMMARY OF INVENTION

This invention describes a novel method of enhancing the potency of DNA rabies vaccines by the addition of small quantities of inactivated virus to the DNA vaccine preparation. In one embodiment, this invention can lead to the development of combination vaccines containing DNA vaccine and inactivated virus. In another embodiment, this invention can lead to the development of vaccines containing much lower quantities of inactivated virus than that present in conventional inactivated virus-based vaccines. This invention also describes a method of producing a novel vaccine composition against rabies virus.

### BRIEF DESCRIPTION OF THE FIGURES:

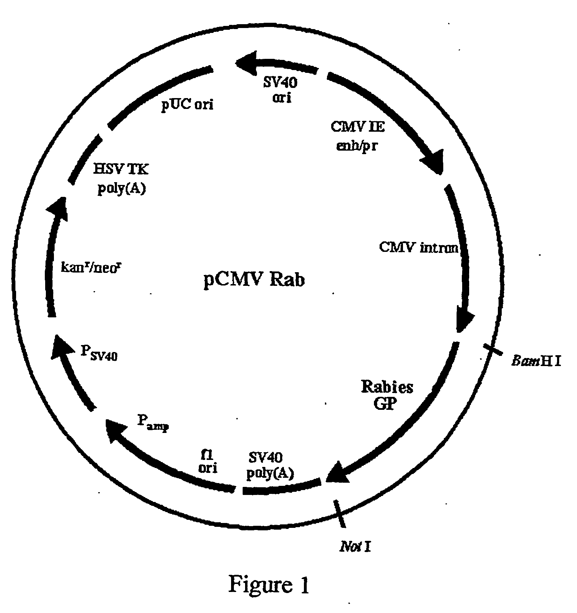

Figure 1 is the schematic representation of the rabies DNA vaccine plasmid (pCMVRab) encoding the rabies virus surface glycoprotein.

### DETAILED DESCRIPTION OF THE INVENTION

The present invention describes a rabies vaccine formulation for use in immunization of vertebrates against rabies using a vaccine formulation comprising a plasmid harbouring DNA sequences encoding surface glycoprotein from rabies virus and inactivated rabies virus. The method described in this invention can be used to develop a combination vaccine consisting mainly of the inactivated virus and plasmid DNA encoding one or more polypeptides of the virus. The vaccine formulation consisting of inactivated rabies virus and DNA vaccine has a much higher potency than that containing inactivated virus alone or DNA vaccine alone, when used at the quantities present in the combination vaccine. As is true for all embodiments of the present invention, the vaccine formulation does not contain live or attenuated viruses.

Potency as described in this invention refers to the ability of the vaccine to induce virus neutralizing antibodies and/or to protect the immunized host against subsequent virus challenge. In one embodiment, the potency of rabies vaccine is evaluated by measuring the rabies virus neutralizing antibody titre in the sera of immunized animals by the rapid fluorescent focus inhibition test or RFFIT (137). In another embodiment, the potency of the rabies vaccine is evaluated by the level of protection conferred by the rabies vaccine in the immunized host against rabies virus challenge.

The inactivated virus-based vaccines described in this invention can be produced by cell/tissue culture methods using any of the vertebrate cell lines known in the prior art. For example, inactivated rabies virus can be produced using vertebrate cells in culture as described in US patents 3423505, 3585266, 4040904, 3769415, 4115195, 3397267, 4664912, 4726946. In one embodiment, a purified chick embryo cell (PCEC) rabies vaccine produced from chick embryo cells were used in combination with rabies DNA vaccine. In another embodiment, purified Vero cell rabies vaccine (PVRV) produced from Vero cells were used in combination with rabies DNA vaccine. In yet another embodiment, rabies virus produced from baby hamster kidney (BHK) cells were used as the source of inactivated rabies virus. Further, the methods of the present invention are considered appropriate for the immunization of vertebrate species such as murine, canine, ovine or humans. In one embodiment, the murine species is a mouse. In another embodiment the canine species is a dog. In yet another embodiment, the bovine species is cattle.

The quantity of inactivated virus in the combination vaccine can vary widely depending on the immunogenicity and potency of the inactivated virus-based formulations. In one embodiment, the quantity of inactivated rabies virus in the combination vaccine is six hundred times less than that present in the tissue culture rabies vaccines known in the prior art. The amount of inactivated rabies virus to be used in combination with rabies DNA vaccine was determined based on careful titration of different dilutions of the inactivated rabies virus vaccine. The dose which failed to induce appreciable levels of VNA in the immunized host and/or failed to confer 100% protection following rabies virus challenge was chosen as the dose to be used in combination with DNA vaccine to develop a combination vaccine with higher potency. It is well known to those skilled in the art of producing rabies vaccines that the final concentration of the inactivated rabies virus and plasmid DNA to be present in the vaccine formulation should be determined by protocols described in the prior art such as those mentioned in U.S. or British phannacopoea using the vaccine standards obtained from World Health Organization (WHO).

As used in this invention, the plasmid DNA refers to extrachromosomal, covalently closed circular DNA molecules capable of autonomous replication in bacterial cells and consist of transcription units (i.e., nucleotide sequences encoding a polypeptides) similar or identical to those present in the virus of interest which are operably linked to transcriptional and translational regulatory sequences necessary for expression of the proteins in the cells of vertebrates.

The transcription unit of the plasmid described in the present invention may contain any of the large number of known eukaryotic promoters such as those found in the genomes of animal viruses and animals including humans. Further, the transcription unit may contain DNA encoding polypeptides of vertebrate pathogens such as viruses, bacteria, parasites etc. The transcription unit consists of DNA encoding the surface glycoprotein of rabies virus.

The introduction of DNA encoding polypeptides of vertebrate pathogens into the plasmids can be carried out by any of the known protocols described in the prior art (138). Further, methods of introducing such plasmids into bacteria, culturing in nutrient media and purification of the plasmids from bacterial cultures can be carried out using any of the processes known in the prior art (138,139).

Examples illustrating the various aspects of this invention are provided below. These examples are only illustrative and not limitative of the remainder of the disclosure in any way whatsoever.

### Example 1: Rabies DNA vaccine preparation and purification.

The rabies DNA vaccine plasmid was constructed as follows. The cytomegalovirus immediate early promoter and intron was isolated from the pCMVintBL plasmid (140) by digestion with the restriction enzymes HindIII and PstI and cloned at the HindIII and PstI sites of the pEGFPI plasmid (Clontech, CA, USA) to obtain pCMVEGFP construct. The cDNA encoding rabies virus surface glycoprotein was isolated as a BgIII restriction fragment from ptg155 vector (141) and cloned into the BamHI site of pCMVEGFP. Finally, the cDNA encoding EGFP was excised out as a XbaI-NotI fragment and the vector was re-ligated to obtain pCMVRab construct. Standard recombinant DNA techniques described in the prior art (138) were used in the construction of pCMVRab. The schematic diagram of pCMVRab is shown in Figure 1. Large scale isolation and purification of pCMVRab were carried out by the alkaline lysis procedure (138,139). The final plasmid preparation was dissolved in saline (0.15M NaCl) and used as rabies DNA vaccine. It is well known to those skilled in the art of preparing eukaryotic expression plasmids that several variations of the above protocol can be used for the development of rabies DNA vaccine plasmid. For example, the cDNA encoding rabies glycoprotein can be derived from other rabies virus strains such as Challenge Virus Standard (CVS), Street-Alabama-Dufferin (SAD), Evelyn Rokitniki Abelseth (ERA) etc. Similarly, the cytomegalovirus promoter can be obtained from several eukaryotic expression plasmids that are available commercially.

### Example 2: Preparation of inactivated rabies virus vaccine from tissue culture of vertebrate cells.

The inactivated rabies virus vaccine can be prepared by any of the methods disclosed in the prior art such as those described in US patents 3423505, 3585266, 4040904, 3769415, 4115195, 3397267, 4664912, 4726946. Briefly, vertebrate cells such as the Vero cells, baby hamster kidney (BHK) cells etc., are infected with rabies virus. The virus is separated from the cellular debris by filtration and then inactivated by the addition of beta-propiolactone or bromoethyleneimine. The virus is then concentrated and a portion is tested for freedom from infectious virus by inoculation of sensitive monolayer cell cultures and by intracerebral inoculation into mice. The concentrated virus preparation is mixed with adjuvants such as aluminium hydroxide (3 milligrams per dose) and the liquid vaccine is used for inoculation of animals. Alternatively, the virus is purified further by zonal centrifugation followed by lyophilization in presence of compounds such as human serum albumin, maltose etc., and the lyophilized preparation is used as rabies vaccine, In one embodiment, the Purifed Vero cell derived rabies vaccine (PVRV) produced by Indian Immunologicals Ltd., Hyderabad, India and known as Abhayrab^{R} was used as the source of inactivated rabies virus vaccine. In another embodiment, the purified chick embryo cell (PCEC) cell derived rabies vaccine produced by Hoechst Marion Roussel, India and known as Rabipur^{R} was used as the source of inactivated rabies virus vaccine. In another embodiment, the veterinery rabies virus vaccine produced from baby Hamster Kidney (BHK) cells by Indian Immunologicals Ltd., Hyderabad, India known as Raksharab^{R} was used as the source of inactivated rabies virus vaccine.

### Example 3: Preparation of combination vaccine consisting of inactivated rabies virus and rabies DNA vaccine.

The inactivated rabies virus vaccines such as Abhayrab^{R}, Raksharab^{R} or Rabipur^{R} were diluted 625 fold with saline and 0.5 ml of the diluted sample was mixed with 100 micrograms of rabies DNA vaccine and the mixture was used as combination rabies vaccine for immunization of mice, dogs or cattle. When necessary, aluminium hydroxide was added to a final concentration of 3.0 milligrams per dose. The final vaccine preparation may also contain preservatives such as Thiomersol (0.015%) and can be used either as a liquid vaccine or as a lyophilized preparation.

### Example 4: Potency of rabies DNA vaccine, inactivated rabies virus vaccine and the combination vaccine consisting of inactivated rabies virus and DNA vaccine as evaluated in a murine rabies virus challenge model.

The potency of rabies DNA was evaluated in a murine peripheral rabies virus challenge model. A group of ten mice were inoculated with 100 micrograms of rabies DNA vaccine per mice twice at an interval of two weeks. Two weeks after the administration of the second dose, mice were inoculated in the foot pads with virulent rabies virus of the challenge virus standard (CVS) strain and observed for 14 days. The results presented in table 1 indicate that only 80% of the mice inoculated with rabies DNA vaccine are protected from rabies virus challenge. We therefore examined whether addition of small quantities of inactivated rabies virus to the rabies DNA vaccine preparation can lead to higher levels of protection. Before addition of inactivated rabies virus to the DNA vaccine, we examined the potency of Abhayrab^{R}, an inactivated rabies virus vaccine produced from Vero cells in the murine rabies virus challenge model. Two hundred microlitres of saline was added to each vial of Abhayrab^{R} (>2.5 IU) and each mouse in a group of ten mice was injected intramuscularly with 0.1 ml of the vaccine. Five fold dilutions of Abhayrab^{R} (1:5, 1:25, 1:125, 1:625) were also prepared and 0.1 ml of each diluted Abhayrab^{R} preparation was injected per mouse by intraperitoneal route. The immunizations were repeated 14 days later. The mice were inoculated in the foot pads with virulent rabies virus of the CVS strain two weeks after administration of the second dose and observed for 14 days. The results presented in Table 1 indicate that undiluted Abhayrab^{R} vaccine as well as that diluted 5, 25 or 125 fold confers 100% protection against rabies virus challenge. Thus, these dilutions were not used in the combination with DNA vaccine since the inactivated virus vaccine at these dilutions conferred 100% protection. However, when diluted to 625 fold, the potency of the Abhayrab^{R} was reduced significantly and only 50% of the mice were protected from rabies virus challenge. This dose of Abhayrab^{R} which confers suboptimal protection was used to study the effect of inactivated rabies virus on the potency of rabies DNA vaccine. The immunization experiments were repeated and mice were inoculated twice at two week interval with 0.1 ml of 625 fold diluted Abhayrab^{R} alone or 100 micrograms of rabies DNA vaccine alone or a combination of both. Mice were challenged two weeks later and the results presented in Table 1 indicates that Abhayrab^{R} and rabies DNA vaccine confer 50% and 80% protection respectively but a combination of these two vaccines results in 100% protection against rabies virus challenge. Thus, addition of suboptimal dose of inactivated rabies virus to rabies DNA vaccine results in the development of a novel combination rabies vaccine with higher potency.

To study whether inactivated rabies virus produced from chick embryo cells can also enhance the potency of rabies DNA vaccine, virus challenge experiments were carried out using Rabipur^{R}, an inactivated rabies virus vaccine produced from chick embryo cells. Two hundred microlitres of saline was added to each vial of Rabipur^{R} (>2.5 IU) and 100 microlitres was diluted upto 625 fold with saline. The diluted vaccine (100 micorlitres) was injected either alone or in combination with rabies DNA vaccine into each mouse twice at two week interval and the mice were challenged two weeks after the last immunization as described above. The results presented in Table 1 indicate that 625 fold diluted Rabipur^{R} confers 60% protection against rabies virus challenge but in combination with rabies DNA vaccine, the level of protection is increased to 100%.

Thus, inactivated rabies virus preparation produced from either Vero cells or chick embryo cells can enhance the potency of rabies DNA vaccine.

### Example 5: Potency of rabies DNA vaccine, inactivated rabies virus vaccine and the combination vaccine consisting of inactivated rabies virus and DNA vaccine as evaluated by RFFIT in a murine model.

The potency of rabies vaccine can also be determined by their ability to induce virus neutralizing antibodies (VNA) in the immunized host as evaluated by the RFFIT (137). Briefly, different dilutions of the serum are incubated with a standard dose of challenge virus preparation in the wells of tissue culture chamber slides and the slides are incubated at 35°C in a humidified carbon dioxide incubator for 60-90 minutes. BHK cells are then added (1 x 10⁵ cells per well) and mixture is incubated as described above for a further 20 hours. The slides are washed in phosphate buffered saline (PBS) and fixed in cold acetone. After drying, fluorescein isothiocyanate-conjugated anti-rabies nucleo protein is added at appropriate dilution and the incubation continued for 20-30 minutes at 37°C. Finally the slides are rinsed in PBS and observed under fluorescent microscope. The virus neutralizing titres are calculated using known protocols. National or international reference serum diluted to a potency of 1.0 IU/ml is used in each test for titrating the titre of the test sera and the results are expressed in terms of IU/ml. A rabies vaccine which induces a VNA titre of 0.5 IU/ml and above is considered to be protective. Thus, the level of VNA in the immunized host is a measure of the rabies vaccine potency and vaccine with better potency induce higher VNA titres. We, therefore, examined whether administration of combination vaccine consisting of 625 fold diluted Abhayrab^{R} and rabies DNA vaccine (100 micrograms) can result in the induction of higher VNA in the immunized animal. Groups of ten mice were inoculated with rabies DNA vaccine alone, different dilutions of Abhayrab^{R} or a combination of 625 fold diluted Abhayrab^{R} and rabies DNA vaccine as described above. Mice were bled by retroorbital puncture before the administration of second dose (day 14 post immunization) as well as two weeks after the administration of the second dose (day 28 post immunization). Sera samples of mice in each group were pooled and the level of rabies VNA was examined by RFFIT. The results presented in Table 2 indicates that 14 days after the administration of a single dose of rabies DNA vaccine alone, 1/625 fold diluted Abhayrab^{R} or both results in the a VNA titre (IU/ml) of 0.287, 0.095 and 1.42 respectively. When the sera were analysed two weeks after the administration of the second dose, the VNA titre (IU/ml) in mice immunized with rabies DNA vaccine alone, 1/625 fold diluted Abhayrab^{R} or both has increased to 1.96, 0.55 and 4.07 respectively. These results clearly indicate that a combination of inactivated rabies virus vaccine and rabies DNA vaccine induces higher levels of VNA in mice than either of them alone.

### Example 6: Potency of rabies DNA vaccine, inactivated rabies virus vaccine and the combination vaccine consisting of inactivated rabies virus and DNA vaccine as evaluated by RFFIT in dogs.

To examine whether the results obtained in mice could be reproduced in other vertebrate species, the immunization experiments were repeated in dogs. Dogs of 3-4 months of age, seronegative for rabies VNA were immunized via intramuscular route with 625 fold diluted Abhayrab^{R}, 100 micrograms of rabies DNA vaccine or both, twice at two week interval. Each group consisted one animal. The animals were bled on day 7, 14, 28 and 35 days postimmunization and the rabies VNA titre in the sera was analysed by RFFIT. The results presented in Table 3 clearly indicates that the VNA titre in the animal inoculated with 625 fold diluted Abhayrab^{R} and DNA vaccine is higher at all time points tested than that in animals inoculated with either DNA vaccine alone or 625 fold diluted Abbayrab^{R} alone.

Thus, inoculation of a combination of inactivated rabies virus and rabies DNA vaccine induces higher levels of rabies VNA not only in mice but also in dogs.

### Example 7: Potency of rabies DNA vaccine, inactivated rabies virus vaccine and the combination vaccine consisting of inactivated rabies virus and DNA vaccine as evaluated by RFFTT in Cattle.

Having demonstrated the potency of the combination vaccine consisting of inactivated rabies virus and rabies DNA vaccine in mice and dogs, we then evaluated its immunogenicity in a large animal model, the cattle. In this experiment, Raksharab^{R}, the inactivated veterinery rabies virus vaccine produced from BHK cells as well as Abhayrab^{R} were used. Cross bred calves of 12-16 months of age, seronegative for antibodies against rabies virus, were vaccinated via intramuscular route with 625 fold diluted Abhayrab^{R} or Raksharab^{R} either alone or in combination with 100 micrograms of rabies DNA vaccine twice at two week interval and blood samples were collected on day 7, 14, 21, 28 and 35 days postimmunization. Each group consisted of three cattle and at each time point, the cattle sera in each group were pooled and then subjected to RFFIT analysis. The results presented in Table 4 clearly indicate that cattle inoculated with inactivated rabies virus (either Abhayrab^{R} or Raksharab^{R}) and rabies DNA vaccine have much higher levels of rabies VNA than those immunized with inactivated rabies virus vaccine alone or rabies DNA vaccine alone.

In another independent experiment, the effect of adjuvants such as aluminium hydroxide on the potency of combination vaccine (inactivated rabies virus + DNA vaccine) was evaluated. Cattle were immunized twice at two week interval (day 0 and day 14) with DNA vaccine, 625 fold diluted Raksharab^{R} or both in the presence or absence of aluminium hydroxide. Cattle were bled at regular intervals and the level of VNA was evaluated by RFFIT. The results presented in Table 5 indicate that addition of aluminium hydroxide further enhances the potency of combination rabies vaccine.

These examples describing the use of rabies DNA vaccine in combination with different inactivated rabies virus vaccines should only be regarded as illustrating and not limiting the invention, which is defined by the appended claims.

**TABLE 1**

| **Protection of mice inoculated with rabies DNA vaccine, inactivated rabies virus vaccine (Abhayrab**^{**R**} **or Rabipur**^{**R**}**) or the combination vaccine from rabies virus challenge** | | | | |
|---|---|---|---|---|
| **Animal groups** | **Rabies vaccine** | **Number of mice inoculated** | **Number of mice survived** | **Percent protection** |
| 1. | DNA vaccine | 10 | 8 | 80 |
| 2. | Abhayrab^{R} (undiluted) | 10 | 10 | 100 |
| 3. | Abhayrab^{R} 1:5 dilution | 10 | 10 | 100 |
| 4. | Abhayarab^{R} 1:25 dilution | 10 | 10 | 100 |
| 5. | Abhayrab^{R} 1:125 dilution | 10 | 10 | 100 |
| 6. | Abhayarab^{R} 1:625 dilution | 10 | 5 | 50 |
| 7. | Abhayrab^{R} 1: 625 dilution + DNA vaccine | 10 | 10 | 100 |
| 8. | Rabipur 1:625 dilution | 10 | 6 | 60 |
| 9. | Rabipur 1:625 dilution + DNA vaccine | 10 | 10 | 100 |
| 10 | Saline | 10 | 0 | 0 |

**TABLE 2**

| **Induction of rabies virus neutralizing antibodies (VNA) in mice by rabies DNA vaccine, inactivated rabies virus vaccine or combination vaccine.** | | | |
|---|---|---|---|
| **Animal groups** | **Rabies vaccine** | **VNA titre (IU/ml)** | |
| | | **Day 14 postimmunization** | **Day 28 postimmunization** |
| 1. | DNA vaccine | 0.287 | 1.96 |
| 2. | Abhayrab^{R} (undiluted) | 2.46 | 4.65 |
| 3. | Abhayrab^{R} 1:5 dilution | 0.62 | 2.82 |
| 4. | Abhayarab^{R} 1:25 dilution | 0.5 | 2.13 |
| 5. | Abhayrab^{R} 1:125 dilution | 0.19 | 1.0 |
| 6. | Abhayarab^{R} 1:625 dilution | 0.095 | 0.55 |
| 7. | Abhayrab^{R} 1: 625 dilution + DNA vaccine | 1.42 | 4.07 |
| 8. | Rabipur^{R} 1:625 dilution | 0.095 | 0.38 |
| 9. | Rabipur^{R} 1:625 dilution + DNA vaccine | 0.54 | 4.26 |

**TABLE 3**

| **Induction of rabies virus neutralizing antibodies (VNA) in dogs by rabies DNA vaccine, inactivated rabies virus vaccine (Abhayrab**^{**R**}**) or the combination vaccine.** | | | | | | | | |
|---|---|---|---|---|---|---|---|---|
| **Animal groups** | **Rabies vaccine** | **Number of dogs** | **VNA titre (IU/ml)**^{**#**} | | | | | |
| | | | **Day 0** | **Day 7** | **Day14** | **Day21** | **Day 28** | **Day 35** |
| 1. | DNA vaccine | 1 | <0.095 | <0.095 | 0.217 | 0.575 | 0.76 | 1.0 |
| 2. | Abhayrab^{R} (1:625 dilution) | 1 | <0.095 | <0.095 | 0.217 | 0.28 | 0.5 | 0.575 |
| 3. | DNA vaccine + Abhayrab^{R} (1:625 dilution) | 1 | <0.095 | <0.095 | 0.575 | 2.0 | 3.02 | 3.02 |

**TABLE 4**

| **Induction of rabies virus neutralizing antibodies (VNA) in cattle by rabies DNA vaccine, inactivated rabies virus vaccine (Abhayrab**^{**R**} **or Raksharab**^{**R**}**) or the combination vaccine.** | | | | | | | | |
|---|---|---|---|---|---|---|---|---|
| **Animal groups** | **Rabies vaccine** | **Number of cattle** | **VNA titre (IU/ml)**^{**#**} | | | | | |
| | | | **Day 0** | **Day 7** | **Day14** | **Day21** | **Day 28** | **Day 35** |
| 1. | DNA vaccine | 3 | <0.095 | 0.66 | 0.36 | 0.80 | 0.87 | 0.96 |
| 2. | Abhayrab^{R} (1:625 dilution) | 3 | <0.095 | 0.21 | 0.33 | 0.32 | 0.47 | 0.64 |
| 3. | DNA vaccine + Abhayrab^{R} (1:625 dilution) | 3 | <0.095 | 0.34 | 0.76 | 2.0 | 3.5 | 3.82 |
| 4. | Raksharab^{R} (1:625 dilution) | 3 | <0.095 | 0.14 | 0.21 | 0.31 | 0.54 | 1.06 |
| 5. | DNA vaccine + Raksharab^{R} (1:625 dilution) | 3 | <0.095 | 0.16 | 0.28 | 1.81 | 2.68 | 3.25 |

**TABLE 5**

| **Effect of adjuvants such as aluminium hydroxide on the induction of rabies virus neutralizing antibodies (VNA) in cattle by rabies DNA vaccine, inactivated rabies virus vaccine (Raksharab) or the combination vaccine.** | | | | | | |
|---|---|---|---|---|---|---|
| **Animal groups** | **Rabies vaccine** | **Number of cattle** | **VNA titre (IU/ml)** | | | |
| | | | **Day 0** | **Day 14** | **Day 21** | **Day 60** |
| 1. | DNA vaccine | 4 | <0.095 | 0.09 | 0.29 | 0.39 |
| 2. | Raksharab^{R} (1:625 dilution) | 4 | <0.095 | 0.13 | 0.30 | 0.11 |
| 3. | DNA vaccine + Raksharab^{R} (1:625 dilution) | 4 | <0.095 | 0.56 | 1.28 | 1.37 |
| 4. | Raksharab^{R} (1:625 dilution) + aluminium hydroxide | 4 | <0.095 | 0.20 | 0.79 | 0.49 |
| 5. | DNA vaccine + Raksharab^{R} (1:625 dilution) + aluminium hydroxide | 4 | <0.095 | 0.79 | 2.12 | 1.88 |

### References:

1. Wolff JA, Malone RW, Williams P, Chong W, Acsadi G, Jani A, Feigner PL. 1990. Direct gene transfer into mouse muscle in vivo. *Science* 247:1465-68.
2. Tang DC, De Vit M, Johnston SA. 1992. Genetic immunization is a simple method for eliciting an immune response. *Nature* 356:152-54.
3. Ulmer JB, Donnelly JJ, Parker SE, Rhodes GH, Felgner PL, Dwarki VJ, Gromkowski SH, Deck RR, De Witt DM, Friedman A, Hawe LA, Leander KR, Martinez D, Perry HC, Shiver JW, Montgomery DC, Liu MA. 1993. Heterologous protection against influenza by injection of DNA encoding a viral protein. *Science* 259:1745-49.
4. Robinson HL, Hunt LA, Webster RG. 1993. Protection against a lethal influenza virus challenge by immunization with a haemagglutinin-expressing plasmid DNA. *Vaccine* 11:957-60.
5. DNA Vaccines: Methods and Protocols. Robert G. Whalen,Douglas B. Lowrie (Editor) / Hardcover / Humana Press / August 1999.
6. Gene Vaccination: Theory and Practice. Eyal Raz (Editor) / Hardcover /Springer-Verlag New York, Incorporated / July 1998.
7. DNA Vaccines: A New Era in Vaccinology. Margaret A. Liu, Reinhard Kurth (Editor),Maurice R. Hilleman (Editor) / Paperback / New York Academy of Sciences / October 1995.
8. Current Topics in Microbiology and Immunology (vol. 226) entitled : DNA Vaccination /Genetic Vaccination edited by David Weiner and Hilary Koprowski.
9. Lewis PJ, Cox GJ, van Drunen Littel-vanden Hurk S, Babiuk LA. 1997. Polynucleotide vaccines in animals: enhancing and modulating responses. *Vaccine*.
10. Babiuk LA, Lewis J, Suradhat S, Baca-Estrada M, Foldvari M, Babiuk S (1999) Polynucleotide vaccines: potential for inducing immunity in animals J Biotechnol, 73: 131-140.
11. Babiuk LA, Lewis J, van den Hurk S, Braun R (1999) DNA immunization: present and future Adv Vet Med, 41: 163-179.
12. Babiuk LA (1999) Broadening the approaches to developing more effective vaccines Vaccine, 17: 1587-1595.
13. Boyle JS, Barr IG, Lew AM (1999) Strategies for improving responses to DNA vaccines Mol Med, 5: 1-8.
14. Cichutek K (1999) Development and standardisation of DNA vaccines Dev Biol Stand, 100: 119-129.
15. Davis HL (1999) DNA vaccines for prophylactic or therapeutic immunization against hepatitis B virus Mt Sinai J Med, 66: 84-90.
16. Donnelly JJ, Ulmer JB (1999) DNA vaccines for viral diseases Braz J Med Biol Res, 32: 215-222.
17. Encke J, zu Putlitz J, Wands JR (1999) DNA vaccines Intervirology, 42: 117-124.
18. Fomsgaard A (1999) HIV-1 DNA vaccines Immunol Lett, 65: 127-131.
19. Freis PC (1999) DNA vaccines [letter] N Engl J Med, 341: 1623-1624.
20. Gendon Iu Z (1999) Progress in developing viral polynucleotide (DNA) vaccines Vopr Virusol, 44: 148-154.
21. Gorecki DC, Simons JP (1999) The dangers of DNA vaccination [letter] Nat Med, 5: 126.
22. Gursel M, Tunca S, Ozkan M, Ozcengiz G, Alaeddinoglu G (1999) Immunoadjuvant action of plasmid DNA in liposomes Vaccine, 17: 1376-1383.
23. Hasan UA, Abai AM, Harper DR, Wren BW, Morrow WJ (1999) Nucleic acid immunization: concepts and techniques associated with third generation vaccines J Immunol Methods, 229: 1-22.
24. Haynes JR (1999) Genetic vaccines Infect Dis Clin North Am, 13: 11-26.
25. Kowalczyk DW, Ertl HC (1999) Immune responses to DNA vaccines Cell Mol Life Sci, 55: 751-770.
26. Kurane I (1999) Present status of basic studies and development of DNA vaccines Nippon Rinsho, 57: 975-981.
27. Leitner WW, Ying H, Restifo NP (1999) DNA and RNA-based vaccines: principles, progress and prospects Vaccine, 18: 765-777.
28. Lewis PJ, Babiuk LA (1999) DNA vaccines: a review Adv Virus Res, 54: 129-188.
29. Ramsay AJ, Kent SJ, Strugnell RA, Suhrbier A, Thomson SA, Ramshaw IA (1999) Genetic vaccination strategies for enhanced cellular, humoral and mucosal immunity Immunol Rev, 171: 27-44.
30. Sasaki S, Inamura K, Okuda K (1999) Genes that induce immunity-DNA vaccines Microbiol Immunol, 43: 191-200.
31. Seder RA, Gurunathan S (1999) DNA vaccines--designer vaccines for the 21st century N Engl J Med, 341: 277-278.
32. Spiegelberg HL, Raz E (1999) DNA vaccines Allergy, 54: 47-48.
33. Stevenson FK (1999) DNA vaccines against cancer; from genes to therapy Ann Oncol, 10: 1413-1418.
34. Stevenson FK, Link CJ, Jr., Traynor A, Yu H, Corr M (1999) DNA vaccination against multiple myeloma Semin Hematol, 36: 38-42.
35. Tuteja R (1999) DNA vaccines: a ray of hope Crit Rev Biochem Mol Biol, 34: 1-24.
36. Watts AM, Kennedy RC (1999) DNA vaccination strategies against infectious diseases Int J Parasitol, 29: 1149-1163.
37. Weiner DB, Kennedy RC (1999) Genetic vaccines Sci Am, 281: 50-57.
38. Whitton JL, Rodriguez F, Zhang J, Hassett DE (1999) DNA immunization: mechanistic studies Vaccine, 17: 1612-1619.
39. www.dnavaccine.com.
40. U.S. Food and Drug Administration. *Points to Consider*. http://www.fda.gov/cber/points.html.
41. De Rose R, McKenna RV, Cobon G, Tennent J, Zakrzewski H, Gale K, Wood PR, Scheerlinck JP, Willadsen P (1999) Bm86 antigen induces a protective immune response against Boophilus microplus following DNA and protein vaccination in sheep Vet Immunol Immunopathol, 71: 151-160.
42. Okuda K, Xin KO, Tsuji T, Bukawa R, Tanaka S, Koff WC, Tani K, Honma K, Kawamoto S, Hamajima K, Fukushima J. 1997. DNA vaccination followed by macromolecular multicomponent peptide vaccination against HIV-1 induces strong antigen-specific immunity. *Vaccine* 15:1049-56.
43. Barnett SW, Rajasekar S, Legg H, Doe B, Fuller DH, Haynes JR, Walker CM, Steimer KS. 1997. Vaccination with HIV-1 gp120 DNA induces immune responses that are boosted by a recom-binant gp120 protein subunit. *Vaccine* 15:869-73.
44. Letvin NL, Montefiori DC, Yasutomi Y, Perry HC, Davies ME, Lekutis C, Alroy M, Freed DC, Lord CI, Handt LK, Liu MA, Shiver JW. 1997. Potent, protective anti-HIV immune responses generated by bimodal HIV envelope DNA plus protein vaccination. *Proc. Natl. Acad. Sci. USA* 94:9378-83.
45. Fuller DH, Corb MM, Barnett S, Steimer K, Haynes JR. 1997. Enhancement of immunodeficiency virus-specific immune responses in DNA-immunized rhesus macaques, *Vaccine* 15:924-26.
46. Hanke T, Blanchard TJ, Schneider J, Hannan CM, Becker M, Gilbert SC, Hill AV, Smith GL, McMichael A. 1998. Enhancement ofMHC class I-restricted peptide-specific T cell induction by a DNA prime/MVA boost vaccination regime. *Vaccine* 16:439-45.
47. Haddad D, Liljeqvist S, Stahl S, Hausson M, Perlmann P, Ahlborg N, Berzins K (1999) Characterization of antibody responses to a Plasmodium falciparum blood-stage antigen induced by a DNA prime/protein boost immunization protocol Scand J Immunol, 49: 506-514.
48. Fensterle J, Crode L, Hess J, Kaufmann SH (1999) Effective DNA vaccination against listeriosis by prime/boost inoculation with the gene gun J Immunol, 163: 4510-4518.
49. Sedegah M, Jones TR, Kaur M, Hedstrom R, Hobart P, Tine JA, Hoffman SL. 1998. Boosting with recombinant vaccinia increases immunogenicity and protective efficacy of malaria DNA vaccine. *Proc. Natl. Acad Sci. USA* 95:7648-53.
50. Schneider J, Gilbert SC, Blanchard TJ, Hanke T, Robson KJ, Hannan CM, Becker M, Sinden R, Smith GL, Hill AV, 1998. Enhanced immunogenicity for CD8 T cell induction and complete protective efficacy of malaria DNA vaccination by boosting with modified vaccinia virus Ankara. *Nat. Med.* 4:397-402.
51. Kent SJ, Zhao A, Best SJ, Chandler JD, Boyle DB, Ramshaw IA. 1998. Enhanced T-cell immunogenicity and protective efficacy of a human immunodeficiency virus type 1 vaccine regimen consisting of consecutive priming with DNA and boosting with recombinant fowlpox virus. *J. Virol*. 72:10180-88.
52. Xiang ZQ, Pasquini S, Ertl HC (1999) Induction of genital immunity by DNA, priming and intranasal booster immunization with a replication-defective adenoviral recombinant J Immunol, 162: 6716-6723.
53. Robinson HL, Montefiori DC, Johnson RP, Manson KH, Kalish ML, Lifson JD, Rizvi TA, Lu S, Hu SL, Mazzara GP, Panicali DL, Herndon JG, Glickman R, Candido MA, Lydy SL, Wyand MS, McClure HM. 1999. Neutralizing antibody-independent containment of immunodeficiency virus challenges by DNA priming and recombinant pox virus booster immunizations. *Nat. Med*. 5:526-34.
54. Richmond JF, Mustafa F, Lu S, Santoro JC, Weng J, O'Connell M, Fenyo EM, Hurwitz JL, Montefiori DC, Robinson HL. 1997. Screening of HIV-1 Env glycoproteins for the ability to raise neutralizing antibody using DNA immunization and recombinant vaccinia virus boosting. *Virology* 230:265-74.
55. Richmond JF, Lu S, Santoro JC, Weng J, Hu SL, Montefiori DC, Robinson HL. 1998. Studies of the neutralizing activity and avidity of anti-human immunodeficiency virus type 1 Env antibody elicited by DNA priming and protein boosting. *J. Virol*. 72:9092-100.
56. Hanke T, McMichael A (1999) Pre-clinical development of a multi-CTL epitope-based DNA prime MVA boost vaccine for AIDS Immunol Lett, 66: 177-181.
57. Hanke T, Samuel RV, Blanchard TJ, Neumann VC, Allen TM, Boyson JE, Sharpe SA, Cook N, Smith GL, Watkins DI, Cranage MP, McMichael AJ (1999) Effective induction of simian immunodeficiency virus-specific cytotoxic T lymphocytes in macaques by using a multiepitope gene and DNA prime- modified vaccinia virus Ankara boost vaccination regimen J Virol, 73: 7524-7532.
58. Calarota SA, Leandersson AC, Bratt G, Hinkula J, Klinman DM, Weinhold KJ, Sandstrom E, Wahren B (1999) Immune responses in asymptomatic HIV-1-infected patients after HIV-DNA immunization followed by highly active antiretroviral treatment J Immunol.
59. Caver TE, Lockey TD, Srinivas RV, Webster RG, Hurwitz JL (1999) A novel vaccine regimen utilizing DNA, vaccinia virus and protein immunizations for HIV-1 envelope presentation Vaccine, 17: 1567-1572.
60. Bot A, Bot S, Bona C. 1998. Enhanced protection against influenza virus of mice immunized as newborns with a mixture ofplasmids expressing hemagglutinin and nucleoprotein. *Vaccine* 16:1675-82.
61. Kim JJ, Ayyavoo V, Bagarazzi ML, Chattergoon M, Boyer JD, Wang B, Weiner DB. 1997. Development of a multicomponent candidate vaccine for HIV-1. *Vaccine* 15:879-83.
62. Whitton JL, Rodriguez F, Zhang J, Hassett DE. 1999. DNA immunization: mechanistic studies. *Vaccine* 17:1612-19.
63. Ciernik IF, Berzofsky JA, Carbone DP. 1996. Induction of cytotoxic T-lymphocyte and antitumor immunity with DNA vaccines expressing single T-cell epitopes. *J. Immunol*. 156:2369-75.
64. Iwasaki A, Dela Cruz CS, Young AR, Barber BH. 1999. Epitope-specific cytotoxic T lymphocyte induction by mini-gene DNA immunization. *Vaccine* 17:2081-88.
65. Yu Z, Karem KL, Kanangat S, Manickan E, Rouse BT. 1998. Protection by mini-genes:a novel approach of DNA vaccines, *Vaccine* 16:1660-67.
66. Hanke T, Schneider J, Gilbert SC, Hill AV, McMichael A. 1998. DNA multi-CTL epitope vaccines for HIV and *Plasmodium falciparum:* immunogenicity inmice. *Vaccine* 16:426-35.
67. Thomson SA, Sherritt MA, Medveczky J, Elliott SL, Moss DJ, Fernando JG, Brown LE, Suhrbier A. 1998. Delivery of multiple CD8 cytotoxic T cell epitopes by DNA vaccination. *J. Immunol*. 160:1717-23.
68. Suhrbier A. 1997, Multi-epitope DNA vaccines. *Immunol. Cell Biol*. 75:402-8.
69. Wang R, Doolan DL, Charoenvit Y, Hedstrom RC, Gardner NJ, Hobart P, Tine J, Sedegah M, Fallarme V, Sacci JB Jr, Kaur M, Klinman DM, Hoffman SL, Weiss WR. 1998. Simultaneous induction of multiple antigen-specific cytotoxic T lymphocytes in nonhuman primates by immunization with a mixture of four *Plasmodium falciparum* DHA plasmids. *Infect. Immun*. 66:4193-202.
70. Shi YP, Hasnain SE, Sacci JB, Holloway BP, Fujioka H, Kumar N, Wohlhueter R, Hoffman SL, Collins WE, Lal AA. 1999. Immunogenicity and in vitro protective efficacy of a recombinant multistage *Plasmodium falciparum* candidate vaccine. *Proc. Natl. Acad Sci. USA* 96:1615-20.
71. Mossman SP, Pierce CC, Robertson MN, Watson AJ, Montefiori DC, Rabin M, Kuller L, Thompson J, Lynch JB, Morton WR, Benveniste RE, Munn R, Hu SL, Greenberg P, Haigwood NL (1999) Immunization against SIVmne in macaques using multigenic DNA vaccines J Med Primatol, 28: 206-213.
72. Mendoza RB, Cantwell MJ, Kipps TJ. 1997. Immunostimulatory effects of a plasmid expressing CD40 ligand (CD154) on gene immunization. *J. Immunol*. 159:5777-81.
73. Kim JJ, Simbiri KA, Sin JI, Dang K, Oh J, Dentchev T, Lee D, Nottingham LK, Chalian AA, McCallus D, Ciccarelli R, Agadjanyan MG, Weiner DB (1999) Cytokine molecular adjuvants modulate immune responses induced by DNA vaccine constructs for HIV-1 and SIV J Interferon Cytokine Res, 19: 77-84.
74. Corr M, Tighe H, Lee D, Dudler J, Trieu M, Brinson DC, Carson DA. 1997. Co-stimulation provided by DNA immunization enhances antitumor iminunity. *J.Immunol*. 159:4999-5004.
75. Lee AH, Sub YS, Sung YC (1999) DNA inoculations with HIV-1 recombinant genomes that express cytokine genes enhance HIV-1 specific immune responses Vaccine, 17: 473-479.
76. Kim JJ, Bagarazzi ML, Trivedi N, Hu Y, Kazahaya K, Wilson DM, Ciccarelli R, Chattergoon MA, Dang K, Mahalingam S, Chalian AA, Agadjanyan MG, Boyer JD, Wang B, Weiner DB. 1997. Engineering of in vivo immune responses to DNA immunization via codelivery of costimulatory molecule genes, *Nat. Bio-technol*. 15:641-46.
77. Tsuji T, Hamajima K, Ishii N, Aoki I, Fukushima J, Xin KQ, Kawamoto S, Sasaki S, Matsunaga K, Ishigatsubo Y, Tani K, Okubo T, Okuda K. 1997. Immunomodulatory effects of a plasmid expressing B7-2 on human immunodeficiency virus-1-specific cell- mediated immunity induced by a plasmid encoding the viral antigen. *Eur. J. Immunol*. 27:782-87.
78. Xiang Z, Ertl HC. 1995. Manipulation of the immune response to a plasmid-encoded viral antigen by coinoculation with plasmids expressing eytokines. *Immunity* 2:129-35.
79. Lee AH, Suh YS, Sung YC. 1999. DNA inoculations with HIV-1 recombinant genomes that express cytokine genes enhance HIV-1 specific immune responses. *Vaccine* 17:473-79.
80. Weiss WR, Ishii KJ, Hedstrom RC, Sedegah M, Ichino M, Barnhart K, Klinman DM, Hoffman SL. 1998. A plasmid encoding murine granulocyte-macrophage colony-stimulating factor increases protection conferred by a malaria DNA vaccine. *J. Immunol*. 161:2325-32.
81. Svanholm C, Lowenadler B, Wigzell H. 1997. Amplification of T-cell and anti-body responses in DNA-based immunization with HIV-1 Nef by co-injection with a GM-CSF expression vector. *Scand. J. Immunol*. 46:298-303.
82. Tsuji T, Hamajima K, Fukushima J, Xin KQ, Ishii N, Aoki I, Ishigatsubo Y, Tani K, Kawamoto S, Nitta Y, Miyazaki J, Koff WC, Okubo T, Okuda K. 1997. Enhancement of cell-mediated immunity against HIV-I induced by coinoculation of plasmid-encoded HIV-1 antigen with plasmid expressing IL-12. *J. Immunol*. 158:4008-13.
83. Kim JJ, Ayyavoo V, Bagarazzi ML, Chat-tergoon MA, Dang K, Wang B, Boyer JD, Weiner DB. 1997. In vivo engineering of a cellular immune response by coadministration of IL-12 expression vector with a DNA immunogen. *J. Immu-nol*. 158:816-26.
84. Larsen DL, Dybdahl-Sissoko N, Mc-Gregor MW, Drape R, Neumann V, Swain WF, Lunn DP, Olsen CW. 1998. Coadministration of DNA encoding interleukin-6 and hemagglutinin confers protection from influenza virus challenge in mice. *J. Virol*. 72:1704-8.
85. Kim JJ, Simbiri KA, Sin JI, Dang K, Oh J, Dentchev T, Lee D, Nottingham LK,Chalian AA, McCallus D, Ciccarelli E, Agadjanyan MG, Weiner DB. 1999. Cytokine molecular adjuvants modulate immune responses induced by DNA vaccine constructs for HIV-1 and SIV, *J. Interferon Cytokine Res*. 19:77-84.
86. Chow YH, Chiang BL, Lee YL, Chi WK, Lin WC, Chen YT, Tao MH. 1998. Development of Th1 and Th2 populations and the nature of immune responses to hepatitis B virus DNA vaccines can be modulated by codelivery of various cytokine genes, *J. Immunol*. 160:1320-29.
87. Geissler M, Gesien A, Tokushige K, Wands JR. 1997. Enhancement of cellular and humoral immune responses to hepatitis C virus core protein using DNA-based vaccines augmented with cytokine-expressing plasmids. *J. Immunol*. 158:1231-37.
88. Chow YH, Huang WL, Chi WK, Chu YD, Tao MH. 1997. Improvement of hepatitis B virus DNA vaccines by plasmids coexpressing hepatitis B surface antigen and interleukin-2. *J. Virol*. 71:169-78.
89. Kim JJ, Trivedi NN, Nottingham LK, Morrison L, Tsai A, Hu Y, Mahalingam S, Dang K, Ahn L, Doyle NK, Wilson DM, Chattergoon MA, Chalian AA, Boyer JD, Agadjanyan MG, Weiner DB, 1998. Modulation of amplitude and direction of in vivo immune responses by co-administration of cytokine gene expression cassettes with DNA immunogens. *Eur. J. Immunol*. 28:1089-103.
90. Gurunathan S, Irvine KR, Wu CY, Cohen JI, Thomas E, Prussin C, Restifo NP, Seder RA. 1998. CD40 ligand/trimer DNA enhances both humoral and cellular immune responses and induces protective immunity to infectious and tumor challenge. *J. Immunol*. 161:4563-71.
91. Maecker HT, Umetsu DT, DeKruyff RH, Levy S. 1997. DNA vaccination with cytokine fusion constructs biases the immune response to ovalbumin. *Vaccine* 15:1687-96.
92. Hakim I, Levy S, Levy R. 1996. A nine amino acid peptide from IL-1β augments antitumor immune responses induced by protein and DNA vaccines. *J. Immunol*. 157:5503-11.
93. Ishii KJ, Weiss WR, Ichino D, Verthelyi D, Klinman DM. 1999. Activity and safety of DNA plasmids encoding IL-4 and IFN-c. *Gene Ther*. 6:237-44.
94. Okada E, Sasaki S, Ishii N, Aoki I, Yasuda T, Nishioka K, Fukushirua J, Miyazaki J, Wahren B, Okuda K. 1997. Intranasal immunization of a DNA vaccine with IL-12- and granulocyte-macrophage colony stimulating factor (GM-CSF) expressing plasmids in lipo-somes induces strong mucosal and cell-mediated immune responses against HIV-1 antigens. *J. Immunol*. 159:3638-47.
95. Iwasaki A, Stiernholm BJ, Chan AK, Berinstein NL, Barber BH. 1997. Enhanced CTL responses mediated by plasmid DNA immunogens encoding costimulatory molecules and cytokines. *J. Immunol*. 158:4591-601.
96. Operschall E, Schuh T, Heinzerling L, Pavlovic J, Moelling K (1999) Enhanced protection against viral infection by co-administration of plasmid DNA coding for viral antigen and cytokines in mice J Clin Virol, 13: 17-27.
97. Ishii N, Tsuji T, Xin KQ, Mohri H, Okuda K. 1998. Adjuvant effect of Uben-imex on a DNA vaccine for HIV-1. *Clin. Exp. Immunol*. 111:30-35.
98. Sasaki S, Sumino K, Hamajima K, Fukushima J, Ishii N, Kawamoto S, Mohri H, Kensil CR, Okuda K. 1998. Induction of systemic and mucosal immune responses to human immunodeficiency virus type 1 by a DNA vaccine formulated with QS-21 saponin adjuvant via intramuscular and intranasal routes. *J. Virol*. 72:4931-39.
99. Sasaki S, Hamajima K, Fukushima J, Ihata A, Ishii N, Gorai I, Hirahara F. Mohri H, Okuda K. 1998. Comparison of intranasal and intramuscular immunization against human immunodeficiency virus type 1 with a DNA-monophosphoryl lipid A adjuvant vaccine. *Infect. Immun*. 66:823-26.
100. Roy K, Mao HQ, Huang SK, Leong KW. 1999. Oral gene delivery with chitosan-DNA nanoparticles generates immunologic protection in a murine model of peanut allergy. *Nat. Med*. 5:387-91.
101. Klavinskis LS, Gao L, Barnfield C, Lehner T, Parker S. 1997. Mucosal immunization with DNA-liposome complexes. *Vaccine* 15:818-20.
102. Haigwood NL, Pierce CC, Robertson MN, Watson AJ, Montefiori DC, Rabin M, Lynch JB, Kuller L, Thompson J, Morton WR, Benveniste RE, Hu SL, Greenberg P, Mossman SP (1999) Protection from pathogenic SIV challenge using multigenic DNA vaccines Immunol Lett, 66: 183-188.
103. Herrmann JE, Chen SC, Jones DH, Tin-sley-Bown A, Fynan EF, Greenberg HB, Farrar GH. 1999. Immune responses and protection obtained by oral immunization with rotavirus VP4 and VP7 DNA vaccines encapsulated in microparticles. *Virology* 259:148-53.
104. Chen SC, Jones DH, Fynan EF, Farrar GH, Clegg JC, Greenberg HB, Hemnann JE. 1998. Protective immunity induced by oral immunization with a rotavirus DNA vaccine encapsulated in microparticles. *J. Virol*. 72:5757-61.
105. Jones DH, Corris S, McDonald S, Clegg JC, Farrar GH. 1997. Poly(DL-lactide-co- glycolide)-encapsulated plasmid DNA elicits systemic and mucosal antibody responses to encoded protein after oral administration. *Vaccine* 15:814-17.
106. Lodmell DL, Ray NB, Ulrich JT, Ewalt LC (2000) DNA vaccination of mice against rabies virus: effects of the route of vaccination and the adjuvant monophosphoryl lipid A (MPL) Vaccine, 18: 1059-1066.
107. Krieg AM (1999) CpG DNA: a novel immunomodulator Trends Microbiol 7.
108. Sato Y, Roman M, Tighe J, Lee D, Corr M, Nguyen MD, Silverman GJ, Lotz M, Carson DA, Raz E, 1996. Immunostimulatory DNA sequences necessary for effective intradermal gene immunization, *Science* 273:352-54.
109. Krieg AM, Yi AK, Matson S, Wald-schmidt TJ, Bishop GA, Teasdale R, Koretzky GA, Klinman DM. 1995. CpG motifs in bacterial DNA trigger direct B-cell activation. *Nature* 374:546-49.
110. Klinman DM, Yamshchikov G, Ishigat-subo Y. 1997. Contribution of CpG motifs to the immunogenicity of DNA vaccines. *J. Immunol*. 158:3635-39.
111. Brazolot Millan CL, Weeratna R, Krieg AM, Siegrist CA, Davis HL. 1998. CpG DNA can induce strong Th1 humoral and cell-mediated immune responses against hepatitis B surface antigen in young mice. *Proc. Natl. Acad Sci. USA* 95:15553-58
112. Klinman DM. 1998. Therapeutic applications of CpG-containing oligodeoxy nucleotides. *Antisense Nucl. Acid Drug Dev*. 8:181-84.
113. Klinman DM, Barnhart KM, Conover J. 1999. CpG motifs as immune adjuvants. *Vaccine* 17:19-25.
114. Zimmermann S, Egeter O, Hausmann S, Lipford GB, Rocken M, Wagner H, Heeg K. 1998. CpG oligodeoxynucleotides trigger protective and curative Th1 responses in lethal murine leishmaniasis. *J. Immunol*. 160:3627-30.
115. Roman M, Martin-Orozco E, Goodman JS, Nguyen MD, Sato Y, Ronaghy A, Kornbluth RS, Richman DD, Carson DA, Raz E. 1997. Immunostimulatory DNA sequences function as T helper-1 promoting adjuvants, *Nat. Med.* 3:849-54.
116. Oxenius A, Martinic MM, Hengartner H, Klenerman P (1999) CpG-containing oligonucleotides are efficient adjuvants for induction of protective antiviral immune responses with T-cell peptide vaccines J Virol, 73: 4120-4126.
117. Paillard F (1999) CpG: the double-edged sword [comment] Hum Gene Ther, 10: 2089-2090.
118. Bryder K, Sbai H, Nielsen HV, Corbet S, Nielsen C, Whalen RG, Fomsgaard A (1999) Improved immunogenicity of HIV-1 epitopes in HBsAg chimeric DNA vaccine plasmids by structural mutations ofHBsAg DNA Cell Biol.
119. Norman JA, Hobart P, Manthorpe M, Felgner P, Wheeler C. 1997. Development of improved vectors for DNA-based immunization and other gene therapy applications. *Vaccine* 15:801-3.
120. Wild J, Gruner B, Metzger K, Kuhrober A, Pudollek HP, Hauser H, Schirmbeck R, Reimann J. 1998. Polyvalent vaccination against hepatitis B surface and core antigen using a dicistronic expression plasmid. *Vaccine* 16:353-60.
121. Uchijima M, Yoshida A, Nagata T, Koide Y. 1998. Optimization of codon usage of plasmid DNA vaccine is required for the effective MHC class I-restricted T cell responses against an intracellular bacterium. *J. Immunol*. 161:5594-99.
122. Andre S, Seed B, Eberle J, Schraut W, Bultmann A, Haas J. 1998. Increased immune response elicited by DNA vaccination with a synthetic gp120 sequence with optimized codon usage. *J. Virol.* 72:1497-503.
123. Vinner L, Nielsen HV, Bryder K, Corbet S, Nielsen C, Fomsgaard A. 1999. Gene gun DNA vaccination with Rev-independent synthetic HIV-1 gp160 envelope gene using mammalian codons. *Vaccine* 17:2166-75.
124. Inchauspe G, Vitvitski L, Major ME, Jung G, Spengler U, Maisonnas M, Trepo C. 1997. Plasmid DNA expressing a secreted or a nonsecreted form of hepatitis C virus nucleocapsid: comparative studies of antibody and T-helper responses following genetic immunization. *DNA Cell Biol*. 16:185-95.
125. Rice J, King CA, Spellerberg MB, Fairweather N, Stevenson FK. 1999. Manipulation of pathogen-derived genes to influence antigen presentation via DNA vaccines. *Vaccine* 17:3030-38.
126. Wu Y, Kipps TJ. 1997. Deoxyribonucleic acid vaccines encoding antigens with rapid proteasome-dependent degradation are highly efficient inducers of cytolytic T lymphocytes. *J. Immunol*. 159:6037-43.
127. Tobery TW, Siliciano RF. 1997. Targeting of HIV-1 antigens for rapid intracellular degradation enhances cytotoxic T lymphocyte (CTL) recognition and the induction of de novo CTL responses in vivo after immunization. *J. Exp. Med*. 185:909-20.
128. Rodriguez F, Zhang J, Whitton JL. 1997. DNA immunization: ubiquitination of a viral protein enhances cytotoxic T-lymphocyte induction and antiviral protection but abrogates antibody induction. *J. Virol*. 71:8497-503.
129. Chaplin PJ, De Rose R, Boyle JS, McWaters P, Kelly J, Tennent JM, Lew AM, Scheerlinck JP (1999) Targeting improves the efficacy of a DNA vaccine against Corynebacterium pseudotuberculosis in sheep Infect Immun, 67: 6434-6438.
130. Forns X, Emerson SU, Tobin GJ, Mushahwar IK, Purcell RH, Bukh J (1999) DNA immunization of mice and macaques with plasmids encoding hepatitis C virus envelope E2 protein expressed intracellularly and on the cell surface Vaccine, 17: 1992-2002.
131. Lewis PJ, van Drunen Littel-van den H, Babiuk LA (1999) Altering the cellular location of an antigen expressed by a DNA-based vaccine modulates the immune response J Virol, 73: 10214-10223.
132. Li Z, Howard A, Kelley C, Delogu G, Collins F, Morris S (1999) Immunogenicity of DNA vaccines expressing tuberculosis proteins fused to tissue plasminogen activator signal sequences Infect Immun, 67: 4780-4786.
133. Lowrie DB, Tascon RE, Bonato VL, Lima VM, Faccioli LH, Stavropoulos E, Colston MJ, Hewinson KG, Moelling K, Silva CL (1999) Therapy of tuberculosis in mice by DNA vaccination Nature, 400: 269-271.
134. Nagata T, Uchijima M, Yoshida A, Kawashima M, Koide Y (1999) Codon optimization effect on translational efficiency of DNA vaccine in mammalian cells: analysis of plasmid DNA encoding a CTL epitope derived from microorganisms Biochem Biophys Res Commun, 261: 445-451.
135. Svanholm C, Bandholtz L, Lobell A, Wigzell H (1999) Enhancement of antibody responses by DNA immunization using expression vectors mediating efficient antigen secretion J Immunol Methods, 228: 121-130.
136. Torres CA, Yang K, Mustafa F, Robinson HL (1999) DNA immunization: effect of secretion of DNA-expressed hemagglutinins on antibody responses Vaccine, 18: 805-814.
137. Smith J S, Yager P A, Baer GM. in Laboratory techniques in rabies, by Meslin F-X, Kaplan M M & Koprowski H, (WHO, Geneva) 1996, 181.
138. Sambrook J, Fritsch EF, Maniatis T. (1989) Molecular cloning; A laboratory Manual, 2^{nd} ed. (Cold Spring Harbour laboratory press, Cold Spring Harbour, NY).
139. Prazeres DMF, Ferreira GNM, Monteiro GA, Cooney CL, Cabral JMS. (1999) large scale production of pharmaceutical-grade plasmid DNA for gene therapy: problems and botlenecks. TIBTECH 17: 169-174.
140. Manthorpe M, Cornefert-Jensen F, Hartikka J, Felgner J, Rundell A, Margalth M, Dwarki VJ. (1993) Gene therapy by intramuscular injection of plasmid DNA: studies on firefly luciferase gene expression in mice. Hum, Gene Ther. 4: 419-421.
141. Keiny, MP, Lathe R, Drillen R, Spehner D, Skory S, Schmitt D, Wiktor T, Koprowski H, Lecocq JP. (1984) Expression of rabies virus glycoprotein from a recombinant vaccinia virus. Nature 312: 163-166.

## Claims

1. A rabies vaccine formulation comprising a plasmid harbouring DNA sequences encoding the surface glycoprotein of rabies virus and inactivated rabies virus.

2. A rabies vaccine as claimed in claim 1, wherein the inactivated virus preparation is produced from vertebrate cells.

3. A rabies vaccine as claimed in claim 2, wherein the vertebrate cells are vero cells, baby hamster kidney cells or chick embryo cells.

4. A process of preparing a rabies vaccine formulation comprising a plasmid harbouring DNA sequences encoding the surface glycoprotein of rabies virus and inactivated rabies virus, which comprises the following steps:
a) Preparing a plasmid construct harbouring DNA sequences encoding the surface glycoprotein of rabies virus;
b) Preparing an inactivated rabies virus preparation;
c) Mixing plasmid construct of step (a) at a dose ranging from 1 to 200 micrograms with inactivated rabies virus preparation of step (b) a buffer between pH 7.0 and 8.0, salts and stabilizers or preservatives;
d) Blending the preparation; and
e) Bottling the preparation.

5. The process of preparing a rabies vaccine formulation as claimed in claim 4 wherein the process further comprises the step of adding adjuvants.

6. The process of preparing a rabies vaccine formulation as claimed in claim 5, wherein the adjuvants comprise aluminium hydroxide.

7. The process of preparing a rabies vaccine formulation as claimed in any one of claims 4 to 6 wherein the salt is sodium chloride.

8. The process of preparing a rabies vaccine formulation as claimed in any one of claims 4 to 7 wherein the stabilizers or preservatives comprise thiomersol, maltose and/or human serum albumin.

9. The process of preparing a rabies vaccine formulation as claimed in any one of claims 4 to 8 wherein the buffers comprise phosphate buffer and/or Tris buffer.

10. Rabies vaccine formulation consisting of plasmid harbouring DNA sequences encoding the surface glycoprotein of rabies virus and inactivated rabies virus for use in immunization of vertebrates against rabies.

11. The rabies vaccine formulation of claim 10 for use in immunization of cattle, dogs or humans.

## Patentansprüche

1. Die Zusammensetzung des Impfstoffes gegen Tollwut ist **dadurch gekennzeichnet, dass** sie ein Plasmid enthält, das die entsprechende DNA-Sequenzen hat, die das Oberflächenglykoprotein von Tollwutviren und inaktivierte Tollwutviren kodieren.

2. Die Zusammensetzung des Impfstoffes nach Anspruch 1 ist **dadurch gekennzeichnet, dass** das Präparat von inaktivierten Tollwutviren aus Wirbeltierzellen hergestellt wird.

3. Die Zusammensetzung des Impfstoffes nach Anspruch 2 ist **dadurch gekennzeichnet, dass** die Wirbeltierzellen Vero Zellen, Zellen der Nieren des Hamstersäuglings, Hühnerembryozellen sind.

4. Das Verfahren der Herstellung der Zusammensetzung des Impfstoffes gegen Tollwut, der ein Plasmid enthält, das die entsprechende DNA-Sequenzen hat, die das Oberflächenglykoprotein von Tollwutviren und inaktivierte Tollwutviren kodieren, ist **dadurch gekennzeichnet, dass** der Prozess folgende Schritte beinhaltet:
a) die Herstellung des Plasmides, das die entsprechende DNA-Sequenzen hat, die Oberflächenglykoprotein von Tollwutviren kodieren;
b) die Herstellung des Präparates von inaktivierten Tollwutviren;
c) die Vermischung des Plasmides, das nach Schritt a) entwickelt wird, mit der Dosis zwischen 1 - 200 Mikrogramm mit inaktivierten Tollwutviren, die nach Schritt b) entwickelt werden, mit einem Puffer, dessen pH-Wert zwischen 7.0 und 8.0 liegt, mit entsprechenden Salzen und stabilisierenden oder konservierenden Stoffen;
d) die Durchmischung der Zusammensetzung;
e) die Abfüllung der Zusammensetzung.

5. Das Verfahren der Herstellung der Zusammensetzung des Impfstoffes gegen Tollwut nach Anspruch 4 ist **dadurch gekennzeichnet, dass** es einen zusätzlichen Schritt gibt, in dem ein Hilfsheilmittel zugegeben wird.

6. Das Verfahren der Herstellung der Zusammensetzung des Impfstoffes gegen Tollwut nach Anspruch 5 ist **dadurch gekennzeichnet, dass** das Hilfsheilmittel Aluminiumhydroxyd enthält.

7. Das Verfahren der Herstellung der Zusammensetzung des Impfstoffes gegen Tollwut nach einem der Ansprüche 4 - 6 ist **dadurch gekennzeichnet, dass** das Salz Natriumchlorid ist.

8. Das Verfahren der Herstellung der Zusammensetzung des Impfstoffes gegen Tollwut nach einem der Ansprüche 4 - 7 ist **dadurch gekennzeichnet, dass** die stabilisierenden oder konservierenden Stoffe Thiomersol, Malzzucker und/oder menschliches Serumalbumin enthalten.

9. Das Verfahren der Herstellung der Zusammensetzung des Impfstoffes gegen Tollwut nach einem der Ansprüche 4 - 8 ist **dadurch gekennzeichnet, dass** die Puffer einen Phosphatpuffer und/oder einen Tris-Puffer enthalten.

10. Die Zusammensetzung des Impfstoffes gegen Tollwut, die ein Plasmid enthält, das die das Oberflächendenglykoprotein von Tollwutviren und inaktivierte Tollwutviren kodierende DNA-Sequenzen hat, zum Gebrauch zur Immunisierung der Wirbeltiere gegen Tollwut

11. Die Zusammensetzung des Impfstoffes gegen Tollwut nach Anspruch 10 zum Gebrauch zur Immunisierung des Viehes, der Hunde oder der Menschen.

## Revendications

1. Formulation vaccinale antirabique comprenant un plasmide hébergeant une séquence d'ADN codant une glycoprotéine de surface du virus de la rage et du virus de la rage inactivé.

2. Un vaccin antirabique selon la revendication 1, dans lequel la préparation du virus de la rage inactivé est produite à partir de cellules de vertébrés.

3. Un vaccin antirabique selon la revendication 2, dans lequel les cellules de vertébrés sont des cellules vero, des cellules de rein d'hamsters nains, des cellules embryonnaires de poulets.

4. Un procédé de préparation de la formulation vaccinale antirabique comprenant un plasmide hébergeant une séquence d'ADN codant une glycoprotéine de surface du virus de la rage et du virus de la rage inactivé qui comprend les étapes suivantes :
a) Préparer une construction d'un plasmide hébergeant une séquence d'ADN codant une glycoprotéine de surface du virus de la rage ;
b) Préparer une préparation du virus de la rage inactivé ;
c) Mélanger la construction du plasmide de l'étape a) à une dose comprise entre 1 et 200 microgrammes avec la préparation du virus de la rage inactivé de l'étape b) , un tampon d'un pH compris entre 7.0 et 8.0, des sels et des stabilisants ou des conservateurs ;
d) Mélanger la préparation; et
e) Embouteiller la préparation.

5. Le procédé de préparation de la formulation vaccinale antirabique selon la revendication 4, dans lequel le procédé comprend en outre une étape d'ajout d'adjuvants.

6. Le procédé de préparation de la formulation vaccinale antirabique selon la revendication 5, dans lequel l'adjuvant comprend un hydroxyde d'aluminium.

7. Le procédé de préparation de la formulation vaccinale antirabique selon l'une quelconque des revendications 4 à 6, dans lequel le sel est le chlorure de sodium.

8. Le procédé de préparation de la formulation vaccinale antirabique selon l'une quelconque des revendications 4 à 7, dans lequel les stabilisants ou les conservateurs comprennent le thiomersol, le maltose et/ou un sérum albumine humain.

9. Le procédé de préparation de la formulation vaccinale antirabique selon l'une quelconque des revendications 4 à 8, dans lequel le tampon comprend un tampon phosphate et/ou un tampon Tris.

10. Une formulation vaccinale antirabique consistant en un plasmide hébergeant une séquence d'ADN codant une glycoprotéine de surface du virus de la rage et du virus de la rage inactivé pour une utilisation dans l'immunisation des vertébrés contre la rage.

11. Une formulation vaccinale antirabique selon la revendication 10 pour une immunisation du bétail, des chiens ou des humains.
